# EUROPEAN PATENT SPECIFICATION

(11) **EP 1 984 057 B1**
(45) Date of publication and mention of the grant of the patent: **24.06.2009**
(21) Application number: 06838632.5
(22) Date of filing: 28.11.2006
(51) Int. Cl.: A61M 25/06

(54) **Enclosed needle device with fluid path access**
Eingeschlossene Nadelvorrichtung mit Flüssigkeitspfadzugang
Dispositif à aiguille enveloppée avec acces par passage de fluide

(30) Priority: 16.02.2006 US 276154
(43) Date of publication of application: 29.10.2008
(73) Proprietor: Smiths Medical ASD, Inc., Rockland, MA 02370-1136 (US)
(72) Inventor: KING, Alan, D., Burlington, CT 06013 (US)
(74) Representative: Findlay, Alice Rosemary
(86) International application number: PCT/US2006/045767
(87) International publication number: WO 2007/094841

(56) References cited:
- EP-A- 1 568 392
- EP-A1- 0 820 783
- EP-A2- 0 734 739
- EP-A2- 1 291 035
- JP-A- 7 328 116
- US-A- 5 695 474

## Description

### Field of the Invention

The present invention relates to needle insertion devices, and more particularly, to an enclosed needle catheter insertion device.

### Description of Prior Art

Over-the-needle catheters are well known in the art. In such devices, a cannula needle projects through a catheter tube with its sharp tip projecting out of the end of the tube. The sharp tip of the needle is used to pierce the skin and the blood vessel so as to carry the end of the catheter into the vessel. Once in place, the needle is withdrawn, leaving the catheter in place for administration or withdrawal of fluids, such as by connection with the now-exposed catheter hub.

In order to reduce the risks of accidental needle sticks after the needle has been removed from the catheter, various proposals have been made to shield the needle tip. One class of devices intended to shield the needle tip includes a needle guard housing into which the needle is received as it is pulled out from the catheter. The guard housing may include as part of its distal end a nose similar to a male slip luer that is adapted to be removably held to the catheter hub. The guard housing is of sufficient length that it essentially encloses the entire length of the needle therein, thus shielding the needle tip. In some cases, the sharp tip is fully inside the guard housing, while in others, the sharp tip may be inside the nose to thus maintain alignment of the needle. In either setting, however, the needle is considered to be enclosed and the tip shielded. To that end, the needle is supported by a needle support hub or housing within the guard housing and which is movable relative to the guard housing from a first position at which the distal end of the support housing is positioned toward the distal end of the guard housing with the needle extending out of the guard housing (and through and out of the catheter when the guard housing is held to the catheter hub), to a second position with the distal end of the support housing positioned away from the distal end of the guard housing so as to withdraw the needle to be enclosed by the guard housing.

The support housing might be spring biased to automatically move the needle into the second position when a latch is activated as shown, for example, in U.S. Patent Nos. 4,747,831 and 5,695,474, and in the commercially available AutoGuard shielded IV catheter from Becton Dickinson and Company. Or the support housing may be manually moved to the second position such as by manipulation of walls or wings of or attached to the support housing. In the manual type of device, a projection and slot detent mechanism on the housings cooperate to retain the housings with the needle in the second position enclosed in the guard housing thus shielding the needle tip. An example of such a manual device is the highly successful PROTECTIV Safety I.V. Catheter marketed by Smiths Medical ASD, Inc., the assignee hereof. After moving into the second, shielded position of the needle, the guard housing may be removed from the catheter hub and discarded with the needle shielded therein, leaving the catheter hub accessible as necessary.

### Summary of the Invention

While devices that use a housing to shield the needle tip by enclosing the needle have been well-accepted and are in widespread use, further improvements are desired. By way of example, in many clinical settings, it is desirable to access the fluid path to the lumen of the needle. Such access is desired for flushing, prior to or during use, or aspiration with a syringe, or for allowing the use of a guidewire during insertion, for example. In commercially available enclosed needle devices such as the AutoGuard or the PROTECTIV devices, however, the needle support housing is quite short in length, sufficient only to support the needle and/or provide a flashback chamber, but otherwise generally confined within the needle guard housing. As a consequence, in the first position, the support housing is recessed well within the guard housing and generally not accessible for attachment of a syringe to flush or aspirate through the needle lumen, or for insertion of a guidewire therein, for example. Further, in the second position, the support housing may still be within the confines of the guard housing and thus still not easily accessible for such purposes.

The present invention provides an enclosed needle catheter insertion device as defined in the independent claim 1 which facilitates fluid or guidewire access to the fluid path to the needle lumen such as to allow for flushing, aspiration, guidewire access, or the like. To that end, and in accordance with the principles of the present invention, the support housing is provided as an elongated housing such that the proximal portion thereof extends out of the guard housing in at least the first position of the needle thereby rendering the proximal portion of the support housing accessible to the medical practitioner. The proximal portion may include or define an access port for use by the medical practitioner to access the fluid path.

Advantageously, the guard and support housings telescope along a common axis, such that the proximal portion of the support housing is accessible at a proximal portion of the guard housing. In that case, the support housing is at least about as long as the guard housing such that the proximal portion will be accessible to the medical practitioner at all times, irrespective of the position of the housing (i.e., whether in the first or second needle positions, or even therebetween).

Further advantageously, a flash plug is associated with the proximal portion of the support housing, and may be removably fitted into the flushing port thereof.

By virtue of the foregoing, there is thus provided an enclosed needle catheter insertion device which facilitates access to the fluid path to the needle lumen. These and other objects and advantages of the present invention shall be made apparent from the accompanying drawings and the description thereof.

### Brief Description of the Drawings

The accompanying drawings, which are incorporated in and constitute a part of this specification, illustrate embodiments of the invention and, together with the general description of the invention given above and the detailed description of the embodiments given below, serve to explain the principles of the present invention.

Fig. 1 is a perspective view of an enclosed needle catheter insertion device which facilitates fluid path access in accordance with the principles of the present invention;

Figs. 2A and 2B are cross-section views of the enclosed needle catheter insertion device of Fig. 1 in respective first and second positions for purposes of explaining the principles of the preset invention;

Figs. 3A and 3B are bottom views, the latter partially cut-away, of the enclosed needle catheter insertion device of Fig. 1 in respective first and second positions for purposes of explaining the principles of the present invention; and

Figs. 4 and 5 are cross-section views of alternative nose and catheter hub arrangements which may be used with the present invention.

### Detailed Description of the Drawings

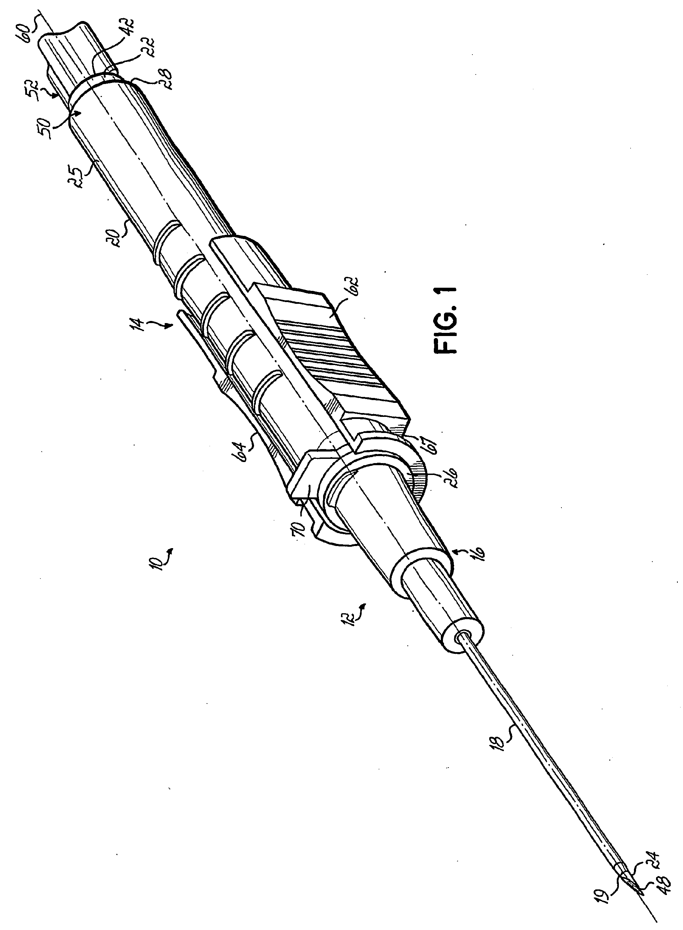

One embodiment 10 of an enclosed needle catheter insertion device in accordance with the principles of the present invention is shown in accompanying Figs. 1 through 3B (which are not necessarily to scale in order to show the various components more readily). Catheter insertion device 10 includes a catheter 12, and a needle insertion assembly 14 including features of the present invention. Catheter 12 includes a catheter hub 16 having an inner wall 17 corresponding to a female luer taper, and a catheter tube 18 held to the catheter hub 16 by any available means (such as an eyelet 15) and extending distally therefrom to its distal, advantageously beveled, end 19. Needle insertion assembly 14 includes an elongated, advantageously cylindrical, outer needle guard housing 20; an elongated, advantageously cylindrical inner needle support chamber or housing 22 telescopingly received in guard housing 20; and a needle 24.

Guard housing 20 has a sidewall 25 extending between distal end or cap 26 and proximal end 28 and defining therewithin a space 30 through which support housing 22 moves and into which needle 24 is ultimately received to be enclosed as will be described below. Distal end 26 includes a nose 32, which may be a male luer taper sized to be removably fitted within the catheter hub 16 and to mate to the inner, female luer taper wall 17 thereof. Housing 20 includes a longitudinal notch 34 running along a portion of its length between ends 26 and 28 for purposes to be described hereinafter.

Support housing 22 has a sidewall 38 extending between distal end 40 and proximal end 42 and defining therebetween a fluid path or lumen 44. Needle 24 is supported by, and may advantageously be affixed to, support housing 22 such that the shaft 46 of needle 24 extends distally from distal end 40 of support housing 22 to a sharp tip 48. The fluid path 44 of support housing 22 is in fluid communication with the lumen 49 of needle 24 such that blood (not shown) may flash back through needle 22 into the fluid path 44 whereby housing 22 also serves as a flash chamber. In that respect, the elongated nature of support housing 22 provides improved flashback visual confirmation. In the embodiment 10 shown herein, housings 20 and 22 are advantageously of about equal length such that the proximal end 42 of support housing 22 is accessible at or beyond the proximal end 28 of guard housing at all times. Support housing 22 could be a bit shorter but still accessible at or beyond proximal end 28 of guard housing 20 due to a cutout (not shown) in guard housing 20 to move proximal end 28 inwardly with a proximal, partial extension (not shown) of guard housing 20 defining an awning or cover (also not shown). A fluid path access port 50 is defined at the proximal end 42 of support housing 22 through which a medical practitioner (not shown) may access fluid path 44 to introduce or withdraw (aspirate) fluids (not shown) through needle lumen 49, such as with a syringe (not shown), or to introduce a guidewire (not shown) therethrough.

To prevent blood (not shown) from exiting support housing 22 during flashback, a flash plug 52 including material 54 adapted to pass air but not blood or other fluids is advantageously associated with support housing 22 near the proximal end 42 thereof. In the embodiment 10 shown herein, flash plug 52 includes a plug housing 56 to hold the material 54 and to define an insert end 58 of male luer taper shape adapted to be frictionally fitted into access port 50 which may advantageously have a mating female luer taper as at 59. Due to the frictional fit therebetween, flash plug 52 normally stays in place closing up flush port 50 against passage of fluids therethrough, but plug 52 can be pulled out giving the medical practitioner access to port 50 for purposes of access to fluid path 44 to needle lumen 49.

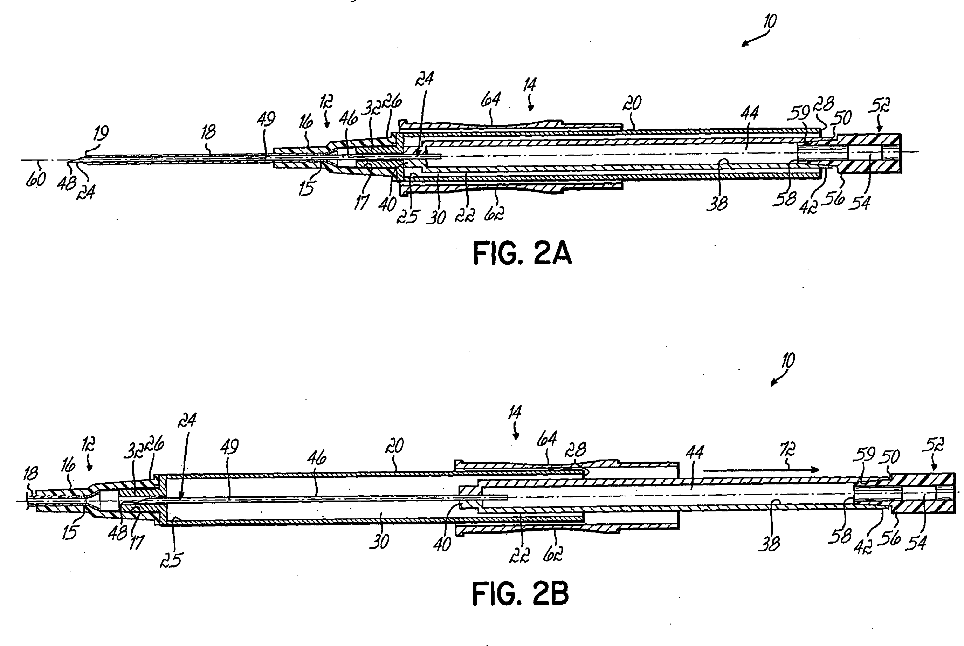

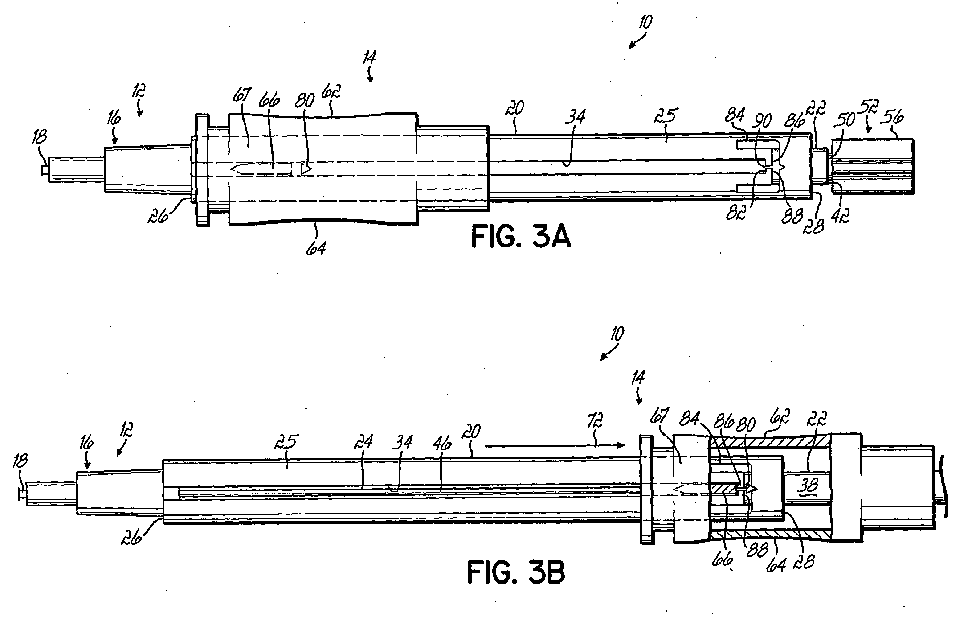

Housings 20 and 22 are telescopingly received such that one may move relative to the other along a common axis 60. To that end, a pair of gripping wings 62, 64 are positioned adjacent but outside of guard housing sidewall 25. A rib 66 extends through lateral notch 34 and joins plate 67 supporting gripping wings 62, 64 to sidewall 38 of support housing 22. A medical practitioner may pull on gripping wings 62, 64 to cause relative motion between housings 20 and 22 from a first position of needle 24 that will be described in connection with Figs. 2A and 3A, to a second position of needle 24 that will be described with reference to Figs. 2B and 3B. The wings 62, 64 may also be joined across the top to, in effect, create a tubular member (not shown) about guard housing 20. The tubular member may be elongated (either as one cylinder or by addition of a cap portion, for example) to match the length of guard housing 20.

In the first position of needle 24, shown in Figs. 2A and 3A, with the distal ends 26 and 40 of housings 20 and 22 positioned towards, and possibly in contact with, each other, needle 24 extends out of the distal end 26 of guard housing 20 and through catheter tube 18, to position sharp needle tip 48 beyond distal end 19 of catheter tube 18. In the first position of needle 24, it will be appreciated that insertion of catheter tube 18 into a patient's blood vessel (not shown) is accomplished by grasping wings 62 and 64 between the thumb and second finger, for example, with the sharp needle tip 48 angled against the patient's skin (not shown) and pushing the entire device distally (to the left in Fig. 2) so as to drive the needle 24 and catheter tube 18 into the patient (not shown). Flashback of blood (not shown) may appear in the flashback chamber defined by fluid path 44 of support housing 22.

Due to the elongated nature of support housing 22 in accordance with the principles of the present invention, proximal end 42 of housing 22 is accessible to the medical practitioner at or beyond proximal end 28 of guard housing 20 in at least the first position shown in Figs. 2A and 3A. Thus, if desired, the medical practitioner (not shown) may, after removal of flash plug 52 (or before it is attached), preflush the device, attach a syringe or load a guidewire (both not shown) prior to insertion. Also, if desired or necessary, during the process of insertion of device 10 and/or after catheter tube distal end 19 is positioned in the vessel (not shown), the medical practitioner (not shown) may remove flash plug 52 and flush or aspirate device 10 through access port 50. Flash plug 52 may then be put back in place when access is no longer required.

After catheter distal end 19 is positioned as desired, needle 24 is to be withdrawn from catheter 12 by causing support housing 22 to move to a second needle position in which the needle shaft 46 and sharp tip end 48 are enclosed by guard housing 20. To this end, the medical practitioner, using one or two hands as desired, pulls on wings 62, 64, possibly by leveraging against push-off tab 70 formed at the distal end 26 of guard housing 20, so as to cause the distal ends 26 and 40 of housings 20 and 22 to move from the position where they are towards each other as seen in Figs. 2A and 3A to a position where they are away from each other (represented by movement of support housing 22 to the right along arrow 72 in Figs. 2B and 3B) as seen in Figs. 2B and 3B. At any time during the traverse from the first to the second position, the medical practitioner (not shown) may access fluid path 44 through access port 50 if desired or necessary. In the second position, needle shaft 46 is within space 30 of guard housing 20 and sharp needle tip 48 is within nose 32, if not all the way into space 30, such that needle 24 is enclosed by guard housing 20.

As the needle 24 moves into the second position, housings 20 and 22 lock into the second needle position by cooperation of lock structure on each of the housings. To that end, a projection 80 coupled to support housing 22 such as on the rib 66 thereof, rides through longitudinal notch 34 of housing 20 until it reaches the proximal end 82 of notch 34. At the proximal end 82 of notch 34, an outer, U-shaped notch 84 is provided to define fingers 86, 88 thereat. Projection 80 is shaped to cam apart the fingers 86, 88 through a slot 90 therebetween, so that projection 80 may pass into U-shaped notch 84, whereafter, fingers 86, 88 snap back together locking projection 80 in place and holding the housings 20 and 22 in the second needle position with needle 24 enclosed within guard housing 20. Guard housing 20 may be removed from catheter 12 by a pulling or twisting motion, leaving hub 16 exposed for use by the medical practitioner. The needle insertion assembly 14 remains in the second needle position and may be discarded.

By virtue of the foregoing, there is thus provided an enclosed needle catheter insertion device which facilitates access to the fluid path to the needle lumen.

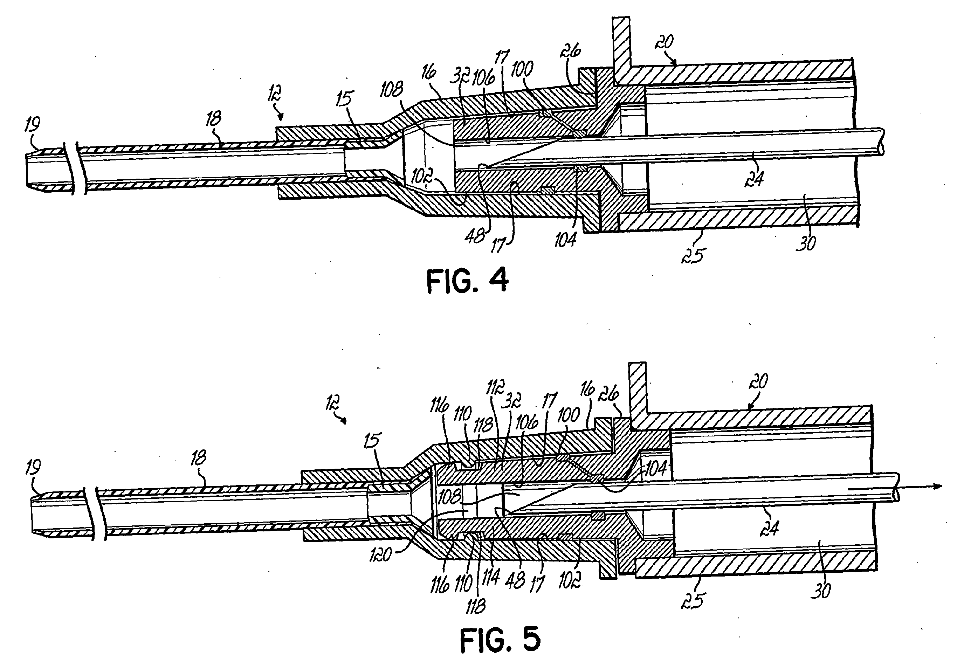

While the present invention has been illustrated by the description of embodiments thereof, and while the embodiments have been described in considerable detail, it is not intended to restrict or in any way limit the scope of the appended claims to such detail. Additional advantages and modifications will readily appear to those skilled in the art. For example, housing 20 and or 22 could be shaped other than cylindrical, including rectangular. Further, the housings 20 and 22 may be held in or moved into the second position of the needle 24 in other ways than as described herein. Nose 32 could include one or more internal or external seals such as co-molded elastomeric gasket 100 integrally associated with exterior wall 102 of nose 32 to form a seal with the inner wall 17 of catheter hub 16 and/or co-molded elastomeric gasket 104 integrally associated with inner wall 106 defining passageway 108 through which needle 24 is received to form a seal therewith as shown in Fig. 4 and as more particularly shown and described in U.S. Patent Application Serial No. 11/276,155 filed February 16, 2006 and entitled "Sealing Catheter Hub Attachment". Further or alternatively, nose 32 and catheter hub 16 could be modified to provide a duckbill release mechanism as shown in U.S. Patent Application Serial No. 11/276,152 filed February 16, 2006 and entitled "Enclosed Needle Device with Duckbill Release Mechanism". To that end, an annular rib 110 (or multiple segments thereof) project from inner wall 17 of hub 16 and a pair of arms 112, 114 extend distally from nose 32 and are adapted to flex. One or both of arms 112, 114 may include a detent 116 defining a recess 118 therebehind with detent 116 normally extending to an outer diametrical distance larger than the inner diameter defined at rib 110 so as to form a releasable hub attachment with rib 110 in recess 118 when needle 24 is not in the space 120 between arms 112 and 114. Nose 32 may also include the integral co-molded elastomeric seals 100 and 104 as described in connection with Fig. 4. Alternatively, although not as desirable, rib 110 could be a recess into wall 17, with detent(s) 116 having a projecting rib on the end of arm 112 and/or 114, and without the recess 118.

## Claims

1. A needle guard for a catheter insertion device comprising an elongated needle guard housing (20) extending between a distal end (26) and a proximal end (28) and defining a space (30) therebetween; an elongated needle support housing (22) telescopingly received within the space (30) of the needle guard housing (20), the needle support housing (22) having a distal end (40) and a proximal end (42) defining a fluid path (44) therebetween; a needle (24) extending distally from the support housing distal end (40) and having a sharp tip (48) extending out beyond the distal end (26) of the guard housing (20) in a first position of the needle (24) with the distal end (40) of the support housing (22) positioned toward the distal end (26) of the guard housing (20), the needle (24) being enclosed by the guard housing (20) in a second position of the needle (24) with the distal end (40) of the support housing (22) positioned away from the distal end (26) of the guard housing (20), the support housing (22) being elongated sufficiently that a proximal portion (42) thereof is accessible at or beyond the proximal end (28) of the guard housing (20) in at least the first position of the needle (24); **characterized by** gripping wings (62, 64) situated outside the guard housing (20) and coupled through a wall of the guard housing (20) to the support housing (22) for manual movement of the needle (24) from the first position to the second position by manipulation of the gripping wings (62, 64).

2. The needle guard as claimed in claim 1, the elongated support housing (22) being spaced closely to the proximal end (28) of the guard housing (20) in at least the first position of the needle (24) so as to provide an elongated flash chamber for improved flashback visual confirmation.

3. The needle guard as claimed in any preceding claim, further comprising a fluid path access port (50) associated with the proximal portion (42) of the support housing (22).

4. The needle guard as claimed in claim 3, the access port (50) being in the proximal portion (42) of the support housing (22).

5. The needle guard as claimed in claim 3, the access port (50) being at the proximal end (42) of the support housing (22).

6. The needle as claimed in any of claims 3 through 5 further comprising a flash plug (52) removably fitted in the access port (50).

7. The needle guard as claimed in any preceding claim, the proximal end (42) of the support housing (22) being accessible.

8. The needle guard as claimed in any preceding claim further comprising cooperating lock structure (80, 90) associated with the guard and support housings (20, 22).

9. The needle guard as claimed in claim 8 wherein the cooperating lock structure includes a projection (80) and a slot (90) associated with respective ones of the needle guard housing (20) and the needle support housing (22).

10. The needle guard as claimed in any preceding claim, the support housing (22) being elongated sufficiently that the proximal portion (42) thereof extends out of the guard housing (22) in at least the first position of the needle.

11. The needle guard as claimed in any preceding claim, the guard housing (20) and the support housing (22) being telescoped along a common axis (60).

12. The needle guard as claimed in any preceding claim, the distal end (26) of the guard housing (22) including a nose (32), the sharp tip (48) of the needle (24) extending beyond the nose (32) in the first position of the needle (24) and being within the nose (32) in the second position of the needle (24).

13. The needle guard as claimed in claim 12, the nose (32) adapted to fit into a catheter hub (16) through which the needle (24) is received, the nose (23) including an elastomeric gasket (100 or 104) integrally associated therewith.

14. The needle guard as claimed in any of claims 1 through 11 in combination with a catheter (12) having a catheter hub (16) and a catheter tube (18) extending distally from the catheter hub (16), the needle (24) extending through the catheter tube (18) such that the sharp tip (48) of the needle (24) is exposed in the first position of the needle (24), the needle (24) being withdrawn from the catheter (12) in the second position of the needle (24).

15. The needle guard as claimed in claim 14, the distal end (26) of the guard housing (22) including a nose (32) fitted into the catheter hub (16) and through which the needle (24) is received.

16. The needle guard as claimed in claim 15, the nose (32) including an elastomeric gasket (100 or 104) integrally associated therewith.

17. The needle guard as claimed in any of claims 13 through 16 further comprising a duckbill release mechanism (110, 116).

## Patentansprüche

1. Kanülenschutz für eine Kathetereinführungsvorrichtung, der Folgendes umfasst: ein längliches Kanülenschutzgehäuse (20), das sich zwischen einem distalen Ende (26) und einem proximalen Ende (28) erstreckt und einen Raum (30) dazwischen definiert; ein längliches Kanülentraggehäuse (22), das in dem Raum (30) des Kanülenschutzgehäuses (20) teleskopartig aufgenommen wird, wobei das Kanülentraggehäuse (22) ein distales Ende (40) und ein proximales Ende (42) hat, wobei dazwischen eine Fluidbahn (44) definiert wird; eine Kanüle (24), die sich distal vom distalen Ende (40) des Traggehäuses erstreckt und eine scharfe Spitze (48) hat, die in einer ersten Position der Kanüle (24) über das distale Ende (26) des Schutzgehäuses (20) hinaus nach außen verläuft, wobei das distale Ende (40) des Traggehäuses (22) in Richtung auf das distale Ende (26) des Schutzgehäuses (20) positioniert ist, wobei die Kanüle (24) in einer zweiten Position der Kanüle (24) vom Schutzgehäuse (20) umschlossen wird, wobei das distale Ende (40) des Traggehäuses (22) vom distalen Ende (26) des Schutzgehäuses (20) weg positioniert ist, wobei das Traggehäuse (22) lang genug ist, damit ein proximaler Abschnitt (42) davon an dem oder jenseits des proximalen Ende(s) (28) des Schutzgehäuses (20) in wenigstens der ersten Position der Kanüle (24) zugänglich ist; **gekennzeichnet durch** Greifflügel (62, 64), die sich auf der Außenseite des Schutzgehäuses (20) befinden und über eine Wand des Schutzgehäuses (20) mit dem Traggehäuse (22) verbunden sind, um die Kanüle (24) **durch** Bedienen der Greifflügel (62, 64) von der ersten Position zur zweiten Position zu bewegen.

2. Kanülenschutz nach Anspruch 1, wobei sich das längliche Traggehäuse (22) in wenigstens der ersten Position der Kanüle (24) nahe dem proximalen Ende (28) des Schutzgehäuses (20) befindet, um eine längliche "Flash"-Kammer für eine verbesserte visuelle "Flashback"-Bestätigung zu erhalten.

3. Kanülenschutz nach einem der vorherigen Ansprüche, der ferner eine Fluidbahnzugangsöffnung (50) umfasst, die mit dem proximalen Abschnitt (42) des Traggehäuses (22) assoziiert ist.

4. Kanülenschutz nach Anspruch 3, wobei sich die Zugangsöffnung (50) im proximalen Abschnitt (42) des Traggehäuses (22) befindet.

5. Kanülenschutz nach Anspruch 3, wobei sich die Zugangsöffnung (50) am proximalen Ende (42) des Traggehäuses (22) befindet.

6. Kanüle nach einem der Ansprüche 3 bis 5, die ferner einen Flash-Stopfen (52) umfasst, der entfernbar in der Zugangsöffnung (50) montiert ist.

7. Kanülenschutz nach einem der vorherigen Ansprüche, wobei das proximale Ende (42) des Traggehäuses (22) zugänglich ist.

8. Kanülenschutz nach einem der vorherigen Ansprüche, der ferner eine kooperierende Verriegelungsstruktur (80, 90) umfasst, die mit dem Schutz- und dem Traggehäuse (20, 22) assoziiert ist.

9. Kanülenschutz nach Anspruch 8, wobei die kooperierende Verriegelungsstruktur ein vorstehendes Teil (80) und einen Schlitz (90) beinhaltet, die jeweils mit dem Kanülenschutzgehäuse (20) und Kanülentraggehäuse (22) assoziiert sind.

10. Kanülenschutz nach einem der vorherigen Ansprüche, wobei das Traggehäuse (22) lang genug ist, damit sich sein proximaler Abschnitt (42) in wenigstens der ersten Position der Kanüle aus dem Schutzgehäuse (22) erstreckt.

11. Kanülenschutz nach einem der vorherigen Ansprüche, wobei das Schutzgehäuse (20) und das Traggehäuse (22) entlang einer gemeinsamen Achse (60) teleskopartig ineinander geschoben werden.

12. Kanülenschutz nach einem der vorherigen Ansprüche, wobei das distale Ende (26) des Schutzgehäuses (22) einen Ansatz (32) beinhaltet, wobei die scharfe Spitze (48) der Kanüle (24) in der ersten Position der Kanüle (24) über den Ansatz (32) hinaus verläuft und sich in der zweiten Position der Kanüle (24) innerhalb des Ansatzes (32) befindet.

13. Kanülenschutz nach Anspruch 12, wobei der Ansatz (32) so gestaltet ist, dass er in eine Katheternabe (16) passt, durch die die Kanüle (24) aufgenommen wird, wobei der Ansatz (23) eine elastomere Dichtung (100 oder 104) beinhaltet, die einstückig damit ausgebildet ist.

14. Kanülenschutz nach einem der Ansprüche 1 bis 11 in Kombination mit einem Katheter (12) mit einer Katheternabe (16) und einer Katheterröhre (18), die sich distal von der Katheternabe (16) erstreckt, wobei die Kanüle (24) so durch die Katheterröhre (18) verläuft, dass die scharfe Spitze (48) der Kanüle (24) in der ersten Position der Kanüle (24) freiliegt, wobei die Kanüle (24) in der zweiten Position der Kanüle (24) aus dem Katheter (12) zurückgezogen wird.

15. Kanülenschutz nach Anspruch 14, wobei das distale Ende (26) des Schutzgehäuses (22) einen Ansatz (32) beinhaltet, der in die Katheternabe (16) eingefügt ist und durch den die Kanüle (24) aufgenommen wird.

16. Kanülenschutz nach Anspruch 15, wobei der Ansatz (32) eine elastomere Dichtung (100 oder 104) beinhaltet, die einstückig damit ausgebildet ist.

17. Kanülenschutz nach einem der Ansprüche 13 bis 16, der ferner einen Entenschnabel-Freigabemechanismus (110, 116) umfasst.

## Revendications

1. Protecteur d'aiguille pour dispositif d'insertion de cathéter comprenant un logement de protecteur d'aiguille allongé (20) qui s'étend entre une extrémité distale (26) et une extrémité proximale (28) et définit un espace (30) entre elles ; un logement de support d'aiguille allongé (22) reçu télescopiquement dans l'espace (30) du logement de protecteur d'aiguille (20), le logement de support d'aiguille (22) ayant une extrémité distale (40) et une extrémité proximale (42) qui définissent un chemin de fluide (44) entre elles ; une aiguille (24) qui s'étend distalement depuis l'extrémité distale (40) du logement de support et ayant un bout pointu (48) qui s'étend au-delà de l'extrémité distale (26) du logement de protecteur (20) à une première position de l'aiguille (24), l'extrémité distale (40) du logement de support (22) étant positionnée vers l'extrémité distale (26) du logement de protecteur (20), l'aiguille (24) étant enfermée par le logement de protecteur (20) à une seconde position de l'aiguille (24), l'extrémité distale (40) du logement de support (22) étant positionnée à l'écart de l'extrémité distale (26) du logement de protecteur (20), le logement de support (22) étant suffisamment allongé pour qu'une partie proximale (42) de celui-ci soit accessible au niveau ou au-delà de l'extrémité proximale (28) du logement de protecteur (20) au moins à la première position de l'aiguille (24) ; **caractérisé par** des ailes de préhension (62, 64) situées en dehors du logement de protecteur (20) et couplées à travers une paroi du logement de protecteur (20) au logement de support (22) en vue d'un mouvement manuel de l'aiguille (24) de la première position à la seconde position par manipulation des ailes de préhension (62, 64).

2. Protecteur d'aiguille selon la revendication 1, le logement de support allongé (22) étant espacé près de l'extrémité proximale (28) du logement de protecteur (20) à au moins la première position de l'aiguille (24) de façon à fournir une chambre transparente allongée pour une meilleure confirmation visuelle du retour.

3. Protecteur d'aiguille selon l'une quelconque des revendications précédentes, comprenant en outre un orifice d'accès au chemin de fluide (50) associé à la partie proximale (42) du logement de support (22).

4. Protecteur d'aiguille selon la revendication 3, l'orifice d'accès (50) se trouvant dans la partie proximale (42) du logement de support (22).

5. Protecteur d'aiguille selon la revendication 3, l'orifice d'accès (50) se trouvant au niveau de l'extrémité proximale (42) du logement de support (22).

6. Aiguille selon l'une quelconque des revendications 3 à 5, comprenant en outre un bouchon de chambre transparente (52) monté de façon amovible dans l'orifice d'accès (50).

7. Protecteur d'aiguille selon l'une quelconque des revendications précédentes, l'extrémité proximale (42) du logement de support (22) étant accessible.

8. Protecteur d'aiguille selon l'une quelconque des revendications précédentes, comprenant en outre une structure de verrou coopérante (80, 90) associée aux logements de protecteur et de support (20, 22).

9. Protecteur d'aiguille selon la revendication 8, dans lequel la structure de verrou coopérante comporte une protubérance (80) et une fente (90) associées à un logement respectif du logement de protecteur d'aiguille (20) et du logement de support d'aiguille (22).

10. Protecteur d'aiguille selon l'une quelconque des revendications précédentes, le logement de support (22) étant suffisamment allongé pour que sa partie proximale (42) sorte du logement de protecteur (22) à au moins la première position de l'aiguille.

11. Protecteur d'aiguille selon l'une quelconque des revendications précédentes, le logement de protecteur (20) et le logement de support (22) étant télescopés le long d'un axe commun (60).

12. Protecteur d'aiguille selon l'une quelconque des revendications précédentes, l'extrémité distale (26) du logement de protecteur (22) comportant un nez (32), le bout pointu (48) de l'aiguille (24) s'étendant au-delà du nez (32) à la première position de l'aiguille (24) et se trouvant à l'intérieur du nez (32) à la seconde position de l'aiguille (24).

13. Protecteur d'aiguille selon la revendication 12, le nez (32) étant adapté pour s'engager dans un embout de cathéter (16) à travers lequel l'aiguille (24) est reçue, le nez (23) comportant un joint élastomère (100 ou 104) faisant partie intégrante de celui-ci.

14. Protecteur d'aiguille selon l'une quelconque des revendications 1 à 11, en combinaison à un cathéter (12) ayant un embout de cathéter (16) et un tube de cathéter (18) qui s'étend distalement depuis l'embout de cathéter (16), l'aiguille (24) s'étendant à travers le tube de cathéter (18) de telle sorte que le bout pointu (48) de l'aiguille (24) soit exposé à la première position de l'aiguille (24), l'aiguille (24) étant rétractée du cathéter (12) à la seconde position de l'aiguille (24).

15. Protecteur d'aiguille selon la revendication 14, l'extrémité distale (26) du logement de protecteur (22) comportant un nez (32) engagé dans l'embout de cathéter (16) et à travers lequel l'aiguille (24) est reçue.

16. Protecteur d'aiguille selon la revendication 15, le nez (32) comportant un joint élastomère (100 ou 104) faisant partie intégrante de celui-ci.

17. Protecteur d'aiguille selon l'une quelconque des revendications 13 à 16, comprenant en outre un mécanisme de détente en bec de canard (110, 116).
